# EUROPEAN PATENT APPLICATION

(11) **EP 2 366 700 A1**
(43) Date of publication of application: **21.09.2011**
(21) Application number: 10386007.8
(22) Date of filing: 19.03.2010
(51) Int. Cl.: C07D 403/06

(54) **Pyrrolidine-thioxopyrimidinone/thiohydantoin organocatolysts**

(71) Applicant: National and Kapodistrian Universty of Athens, 10561 Athens (GR)
(72) Inventor: Kokotos George,, Athens 15561 (GR); Kokotos Christoforos, Athens 15561 (GR); Limnios Dimitrios, Athens 17562 (GR)
(74) Representative: Roukounas, Dimitrios

(57) **Abstract**

Organocatalysts that comprise a pyrrolidine ring and a thiodydantoin or a thioxopyrimidinone ring. The compounds are effective as catalysts in enantioselective and stereoselective organic reactions.

## Description

### FIELD OF THE INVENTION

The present invention relates to novel organocatalysts that facilitate enantioselective and stereoselective organic reactions. These compounds comprise a pyrrolidine ring and a thioxopyrimidinone or a thiohydantoin ring.

### BACKGROUND OF THE INVENTION

Until the end of 20^{th} century, metal complexes and biocatalysts were the major classes of catalysts used in chemical transformations. However, nowadays organocatalysis, that means metal-free catalysis, has been recognized as the most modern field of catalysis allowing the stereoselective formation of various chiral organic molecules (For a book see, Dalko, P. I. Enantioselective Organocatalysis: Reactions and Experimental Procedures; Wiley-VCH: Weinheim, 2007). The major categories of organocatalysts are: proline and proline-based catalysts, chiral thioureas and imidazolidinones. The synthesis and the catalytic activities of the existing organocatalysts are summarized in a number of review articles (see, Dalko and Moisan, Angew. Chem. Int. Ed. 2004, 43, 5138; S. Mukherjee et al, Chem. Rev. 2007, 107, 5471-5569; X. Liu et al, Chem. Commun. 2009, 6145). The various organocatalysts present distinct mechanisms of activation mode. The five-membered secondary amine structure of pyrrolidine is considered as a "privileged" structure able to activate carbonyl compounds through the formation of enamine intermediates. In combination with other functional groups, it provides bifunctional molecules able to catalyze a variety of asymmetric transformations.

The natural amino acid proline is a good catalyst for various enantioselective reactions, like aldol reaction, Michael reaction, Mannich reaction etc. (for examples, List et al, J. Am. Chem. Soc. 2000, 122, 2395; List et al, Org. Lett. 2001, 3, 2423).

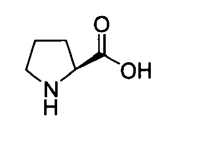

Various prolyl-based sulfonamides present improved catalytic preoperties in comparison to proline (Berkessel et al, Adv. Synth. Catal. 2004, 346, 1141; Bellis et al, Synthesis 2005, 2407; Wang et al, Angew. Chem. Int. Ed. 2005, 44, 1369).

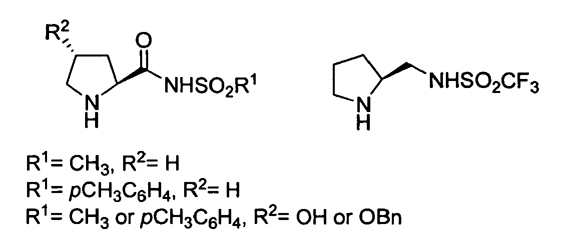

(S)-1-(2-Pyrrolidinylmethyl)pyrrolidine is a diamine showing improved catalytic activity for the asymmetric conjugate addition of ketones to nitroolefins (Betancort et al, Tetrahedron Lett. 2001, 42, 4441)**.**

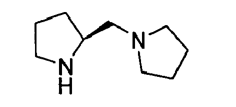

Diarylprolinols and the corresponding silyl ethers constitute another class of pyrrolidine-based organocatalysts (Marigo et al, Angew. Chem. Int. Ed. 2005, 44, 794; Hayashi et al, Angew. Chem. Int. Ed. 2005, 44, 4212).

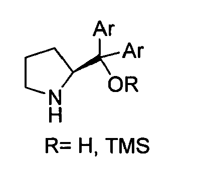

Amides derived from proline and chiral amino alcohols constute another important catalytic class (Tang et al, Proc. Natl. Acad. Sci.USA 2004, 101, 5755).

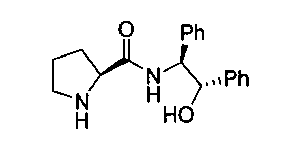

The known organocatalysts have been demonstrated to catalyze efficiently various organic transformations like the aldol reaction, the Michael addition, the α-substitution reaction etc. The primary advantage of organocatalysis is that it avoids the use of metals which can be expensive and toxic. Although some of the known catalysts provide the products of organic transformations in high yield and stereoselectivity, one major drawback exists. Usually, a high catalyst loading (10-30%) has to be applied. Thus, there is a great need for novel highly efficient organocatalysts able to catalyze an asymmetric transformation at low catalyst loading (<5%) providing the product in high yield and selectivity.

### SUMMARY OF THE INVENTION

The present invention provides novel organocatalysts that comprise the secondary amine of the pyrrolidine ring and a thiodydantoin or a thioxopyrimidinone ring.

The compounds of the present invention are effective as catalysts in enantioselective and stereoselective organic reactions and they may be successfully employed at low catalyst loading. Furthermore, they are stable compounds and can be easily synthesized from commercially available starting materials.

### DETAILED DESCRIPTION OF THE INVENTION

The present invention provides the novel organocatalysts of formula (I) 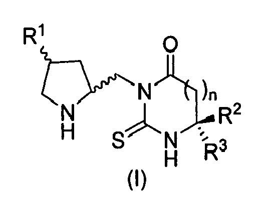 wherein
R¹ is H, OH, O-alkyl, OSi(CH₃)₃, OSiC(CH₃)₃(CH₃)₂,
R² is H, alkyl, aryl,
R³ is H, alkyl, aryl,
provided that when R² is H, CH₃, then R³ is other than H, CH₃,
n is 0 or 1,
their stereoisomers,
and their salts thereof.

The term "alkyl" refers to the radical of saturated aliphatic groups, including straight chain alkyl groups, branched-chain alkyl groups, cycloalkyl (alicyclic) groups, alkyl substituted cycloalkyl groups, and cycloalkyl substituted alkyl groups. Typical alkyl groups include, but are not limited to, methyl, ethyl, propyl, isopropyl, n-butyl, isobutyl, t-butyl, n-pentyl, isopentyl, hexyl, etc.

The term "aryl" as used herein refers to a C₆₋₁₂ monocyclic or bicyclic hydrocarbon ring wherein at least one ring is aromatic. Examples of such groups include phenyl, naphthyl or tetrahydronaphthalenyl and the like.

The compounds described herein contain one or two asymmetric centers and may thus give rise to diastereomers and optical isomers. The present invention is meant to include all such possible diastereomers in enantiomerically pure forms.

Preferable compounds according to the present invention are those where R¹ is H and R², R³ and n are as defined above.

More preferable compounds are those where R¹ is H, one of R² and R³ is H and the other is C2-C5 alkyl, phenyl.

The compounds of the present invention combine the secondary amine of the pyrrolidine ring with a new chiral scaffold, which is the thiohydantoin ring or the thioxopyrimidinone ring. This chiral scaffold is capable of creating one or more hydrogen bonds with the substrate and/or steric hindrance that facilitates the formation of the reaction products in great enantiomeric and diastereomeric excess.

A general method for the synthesis of the new catalysts is depicted in Figure 1. Methyl esters of α- or β-amino acids 1 (which are either commercially available or may by synthesized from commercially available starting materials following methods from the prior art) are converted to the corresponding isothiocyanates by treatment with thiophosgene and react with (S)-tert-butyl-2-(aminomethyl)pyrrolidine-1-carboxylate to produce thioureas **2.** It is known that ureas or thioureas are cyclized to the corresponding hydantoins, thiohydantoins and thioxotetrahydropyrimidinones under acidic or basic conditions (Sim et al, J. Org. Chem. 1997, 62, 9358; V. Kumar, V. A. Nair, Tetrahedron Lett. 2010, 51, 966).

Treatment of thioureas **2** with 6N HCl/MeOH upon heating result in the formation of the heterocyclic ring with simultaneous removal of the Boc group providing compounds **3**.

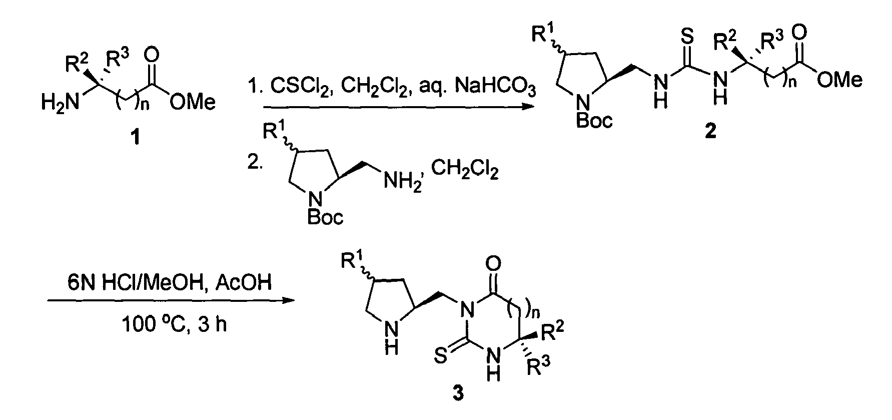

Firgure 1. A general method for the synthesis of new catalysts **3**.

Examples of the new catalysts **3a-g** are compounds prepared starting from (*S*)-β-phenylalanine (**1a**), β-alanine (**1b**), glycine (**1c**) (*S*)-phenylalanine (**1d**), (*S*)-phenylglycine (**1e**), (*S*)-*tert*-leucine (**1f**) and (*R*)-phenylglycine (**1g**).

| **1-3** | n | R¹ | R² | R³ |
|---|---|---|---|---|
| **a** | 1 | H | Ph | H |
| **b** | 1 | H | H | H |
| **c** | 0 | H | H | H |
| **d** | 0 | H | CH₂Ph | H |
| **e** | 0 | H | Ph | H |
| **f** | 0 | H | ^{t}Bu | H |
| **g** | 0 | H | H | Ph |

Thioureas **2** may be also cyclized to compounds **4** by treatment with triethylamine in the presence of LiClO₄ under heating for 2 hours (Figure 2). The Boc protecting group is then removed by treatment with HCl/MeOH at room temperature to receive compounds **3**.

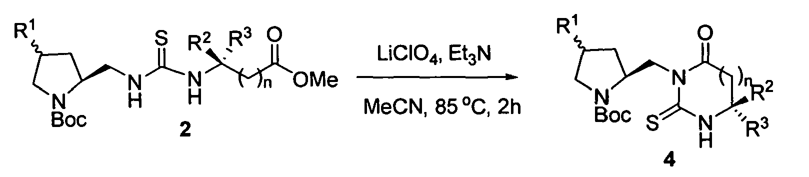

Figure 2. Cyclization of thioureas **2** under basic conditions.

The compounds of the present invention may be used in enantioselective and stereoselective organic reactions, like Michael additions, aldol reactions, Mannich reactions, α-substitution reactions etc. Preferably, they are used in a Michael addition reaction.

The compounds of the present invention may be used in lower catalyst loading than that of other known organocatalysts, which reduces the cost and facilitates the work up of the reactions. The catalyst loading refers to mol% of the catalyst in comparison to the limiting reagent (in the case of Michael reaction, the limiting reagent is the nitroolefin). The compounds of the present invention are advantageously effective in amounts as low as 2.5% or even 1% catalyst loading.

Furthermore, the compounds of the present invention do not require the presence of a big excess of one of the reactants in the reaction mixture and thus they are efficient even when only a small excess of one of the reactants is present.

The present invention is illustrated by the following examples.

### EXAMPLES

### Example 1

Table 1 demonstrates the efficacy of one of the compounds of the present invention in a Michael reaction between cyclohexanone and trans-β-nitrostyrene. The reaction is preferably carried out in the presence of an acid and water as additives.

**Table 1. Michael reaction between cyclohexanone and trans-β-nitrostyrene using catalyst 3a.^{[a]}**

| | | | | | |
|---|---|---|---|---|---|
| 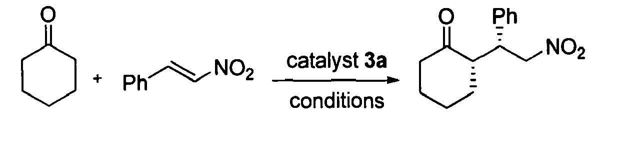 | | | | | |

| Entry | Catalyst loading (%) | Solvent | Additives | Yield (%)^{[b]} | ee (%)^{[c,d]} |
|---|---|---|---|---|---|
| 1 | 2.5 | toluene | AcOH, H₂O | 95 | 92 |
| 2 | 2.5 | THF | AcOH, H₂O | 92 | 96 |
| 3 | 2.5 | toluene | 4-NO₂PhCOOH, H₂O | 96 | 97 |
| 4 | 2.5 | THF | 4-NO₂PhCOOH, H₂O | 100 | 96 |
| 5 | 1 | THF | 4-NO₂PhCOOH, H₂O | 86 | 97 |
| 6^{[e]} | 2.5 | THF | 4-NO₂PhCOOH, H₂O | 98 | 96 |
| 7^{[f]} | 1 | THF | 4-NO₂PhCOOH, H₂O | 71 | 96 |

| | | | | | |
|---|---|---|---|---|---|
| ^{[a]} Reactions were performed using 0.2 mmol nitroolefin, ketone (10 eq), acid (15 mol%) and water (2 eq) for 18 h.^{[b]} Isolated yield after column chromatography.^{[c]} The enantiomeric excess (*ee*) was determined by chiral HPLC. ^{[d]} The diastereomeric ratio (dr) was determined by ¹H NMR spectroscopy and found 99:1. ^{[e]} 2 eq of ketone were used, reaction time 48 h, dr 98:2. ^{[f]} 1.2 eq of ketone were used, reaction time 48 h, dr 98:2. | | | | | |

As it can be seen from table 1, the product is obtained in high yield and enantiomeric excess (ee) even when as little as 1% of the catalyst is used. Furthermore, the compound of the present invention efficiently catalyses the reaction even when a small excess of the ketone is used (entries 6 and 7).

### Example 2

This example demonstrates the efficiency of the compounds of the present invention in a Michael reaction between cyclohexanone and trans-β-nitrostyrene. The reaction is carried out at room temperature in the presence of p-nitrobenzoic acid and water as additives. The catalyst loading was 2.5 mol% and the solvents used were toluene and tetrahydrofuran (THF).

**Table 2. Michael reaction of cyclohexanone with trans-β-nitrostyrene using catalysts 3a-g.^{[a]}**

| | | | | | |
|---|---|---|---|---|---|
| 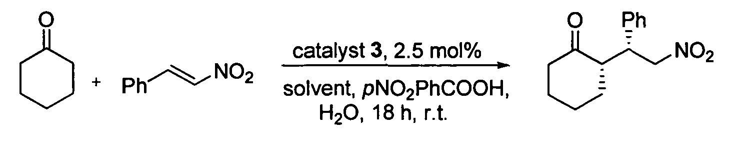 | | | | | |

| Entry | Catalyst | Solvent | Yield (%)^{[b]} | dr^{[c]} | ee (%)^{[d]} |
|---|---|---|---|---|---|
| 1 | **3a** | toluene | 96 | 99:1 | 97 |
| 2 | **3a** | THF | 100 | 99:1 | 96 |
| 3 | **3b** | toluene | - | | |
| 4 | **3b** | THF | - | | |
| 5 | **3c** | THF | 24 | 96:4 | 92 |
| 6 | **3d** | toluene | 92 | 98:2 | 92 |
| 7 | **3e** | toluene | 92 | 99:1 | 95 |
| 8 | **3e** | THF | 96 | 99:1 | 95 |
| 9 | **3f** | toluene | 72 | 99:1 | 95 |
| 10 | **3f** | THF | 100 | 99:1 | 95 |
| 12 | **3g** | toluene | 81 | 99:1 | 94 |
| 13 | **3g** | THF | 91 | 99:1 | 96 |

| | | | | | |
|---|---|---|---|---|---|
| ^{[a]} Reactions were performed using 0.2 mmol nitroolefin, ketone (10 eq), acid (15 mol%) and water (2 eq) for 18 h. ^{[b]} Isolated yield after column chromatography. ^{[c]} The dr was determined by ¹H NMR spectroscopy. ^{[d]}The ee was determined by chiral HPLC. | | | | | |

Compounds **3b** and **3c** provided traces or low yield of the product (entries 3-5, Table 2) indicating the importance of at least one substituent other than hydrogen at the 6-position or 5-position of the thioxopyrimidinone or thiohydantoin ring, respectively. On the other hand, compounds **3d, 3e** and **3f** exhibit excellent yield and ee. Both the thioxo-pyrimidinone ring and the thiohydantoin ring carrying a phenyl substituent work excellent as catalysts for the reaction between the cyclohexanone and *trans-β-*nitrostyrene.

### Example 3

This example demonstrates the efficacy of compound **3a** in a Michael reaction between various nitroolefins and ketones. The reaction was carried out at room temperature in THF, in the presence of p-nitro benzoic acid and water as additives. The catalyst loading was 2.5 mol%

**Table 3.**

| | | | | | | | |
|---|---|---|---|---|---|---|---|
| 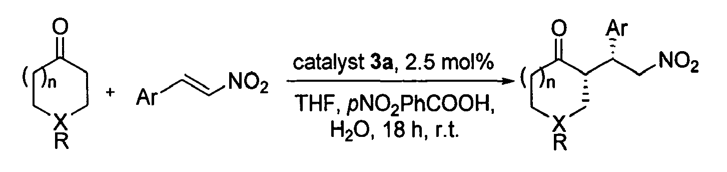 | | | | | | | |

| Entry | X | R | n | Ar | Yield (%)^{[b]} | dr^{[c]} | ee (%)^{[d]} |
|---|---|---|---|---|---|---|---|
| 1 | CH₂ | - | 1 | furyl | 89 | 95:5 | 95 |
| 2 | CH₂ | - | 1 | 4-FPh | 92 | 99:1 | 97 |
| 3 | CH₂ | - | 1 | 4-NO₂Ph | 100 | 97:3 | 97 |
| 4 | CH₂ | - | 1 | naphthyl | 88 | 99:1 | 96 |
| 5 | O | - | 1 | Ph | 98^{[e]} | 93:7 | 95 |
| 6 | S | - | 1 | Ph | 99^{[e]} | 97:3 | 95 |
| 7 | C | (CH₃)₂ | 1 | Ph | 96^{[e]} | 98:2 | 96 |
| 8 | N | Ac | 1 | Ph | 91^{[e]} | 96:4 | 96 |
| 9 | C | 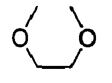 | 1 | Ph | 93^{[e]} | 98:2 | 95 |
| 10 | CH | - | 0 | Ph | 50% | 95:5 | 94 |

| | | | | | | | |
|---|---|---|---|---|---|---|---|
| ^{[a]} Reactions were performed using 0.2 mmol nitroolefin, ketone (10 eq), acid (15 mol%) and water (2 eq) for 18 h. ^{[b]} Isolated yield after column chromatography. ^{[c]} The dr was determined by ¹H NMR spectroscopy. ^{d}The *ee* was determined by chiral HPLC. ^{[e]}reaction time 48-60 h. | | | | | | | |

The reaction between cyclohexanone and various nitroolefins took place with high diastereo- (95:5 to 98:2) and enantio-selectivity (95-97% ee) (entries 1-4, Table 3). The product was isolated in excellent yields (88-100%). When other ketones were used with *trans-*β-nitrostyrene, elongation of the reaction time was needed to reach reaction completion. However, the products were isolated in almost quantitative yield and high selectivities after 2 to 3 days (entries 5-10, Table 3).

The above examples demonstrate that the compounds of the present invention are highly effective in the asymmetric Michael addition reaction while they may be used in amounts as little as 1 mol%.

### Example 4

This example demonstrates the process for the production of the compounds of the present invention.

### A. General procedure for the synthesis of thioureas from amino acids

To a stirring solution of amino acid methyl ester hydrochloride **1** (1.0 mmol) in dichloromethane (5 mL), a saturated aqueous solution of NaHCO₃ (5 mL) was added at 0 °C and left stirring vigorously for 10 min. The stirring was stopped and thiophosgene (0.1 mL, 1.05 mmol) was added to the organic layer (bottom layer) via syringe. The reaction mixture was stirred vigorously at room temperature for 1 h. The two layers were separated and the aqueous layer was extracted with dichloromethane (3 × 5 mL). The combined organic layers were dried over Na₂SO₄ and the solvent was evaporated to afford the isothiocyanate with enough purity to be used in the subsequent step. A solution of the isothiocyanate in dichloromethane (15 mL) was added to a stirring solution of (*S*)-*tert*-butyl-2-(aminomethyl)pyrrolidine-1-carboxylate (0.20 g, 1.0 mmol) in dichloromethane (10 mL) over a period of 5 min at room temperature and the reaction mixture was left stirring until the consumption of the isothiocyanate (0.5-5 h). The solvent was evaporated and the crude product was purified using flash column chromatography eluting with various mixtures of CHCl₃:MeOH or CH₂Cl₂:MeOH.

Following the above process, the following compounds are prepared:

### (S)-tert-Butyl-2-{[3-((S)-3-methoxy-3-oxo-1-phenylpropyl)thioureido]methyl}pyrrolidine-1-carboxylate (2a)

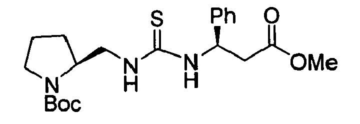

Yield 88%; light yellow solid; mp 64-66 °C; [α]_{D}= -1.5 (*c* = 1.0, CH₂Cl₂); ¹H NMR (200 MHz, CDCl₃) δ 8.93 (1H, br d, *J* = 8.2 Hz, NH), 7.43-7.17 (5H, m, ArH), 6.39 (1H, br s, NH), 5.56 (1H, ddd, *J* = 11.3, 8.2 and 6.1 Hz, NCH), 4.15-3.99 (1H, m, NCH), 3.72 (3H, s, OCH₃), 3.62-3.21 (4H, m, 4 x NCHH), 3.19-3.01 (2H, m, COCHH), 2.01-1.71 (4H, m, 2 x CHH), 1.52 [9H, s, C(CH₃)₃]; ¹³C NMR (50 MHz, CDCl₃) δ 181.3 (181. 5) (C=S), 173.6 (172.4) (C=O), 154.6 (154.2) (OCONH), 135.5 (134.7) (Ar), 129.0 (Ar), 128.1 (Ar), 126.6 (127.0) (Ar), 80.0 [C(CH₃)₃], 60.3 (60.3) (NCH), 55.9 (NCH), 52.0 (OCH₃), 46.5 (46.4) (NCH₂), 46.1 (45.9) (NCH₂), 36.5 (37.7) (CH₂CO), 29.7 (29.7) (CH₂), 28.2 [C(CH₃)], 23.5 (23.2) (CH₂); MS (ESI) 422 (M+H⁺, 100%).

### (S)-tert-Butyl-2-{[3-(3-methoxy-3-oxopropyl)thioureido]methyl}pyrrolidine-1-carboxylate (2b)

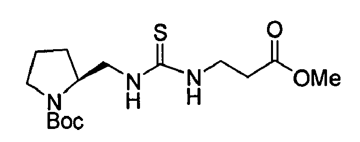

Yield 82%; light yellow oil; [α]_{D}= -8.1 (*c* = 1.0, CH₂Cl₂); ¹H NMR (200 MHz, CDCl₃) δ 7.95 (1H, br s, NH), 6.94 (0.5H, br s, NH), 6.63 (0.5H, br s, NH), 3.95-3.51 (4H, m, NCH and 3 x NCHH), 3.51 (3H, s, OCH₃), 3.37-3.07 (3H, m, 3 x NCHH), 2.54 (2H, t, *J* = 6.2 Hz, COCH₂), 1.89-1.58 (4H, m, 2 x CH₂), 1.29 [9H, s, C(CH₃)₃]; ¹³C NMR (50 MHz, CDCl₃) δ 180.7 (C=S), 171.8 (C=O), 156.4 (OCONH), 79.9 [C(CH₃)₃], 55.8 (NCH), 51.4 (OCH₃), 46.6 (NCH₂), 46.5 (NCH₂), 46.3 (NCH₂), 33.1 (CH₂CO), 29.3 (CH₂), 28.0 [C(CH₃)], 23.4 (CH₂); MS (ESI) 346 (M+H⁺, 100%).

### (S)-tert-Butyl-2-{[3-(2-methoxy-2-oxoethyl)thioureido]methyl}pyrrolidine-1-carboxylate (2c)

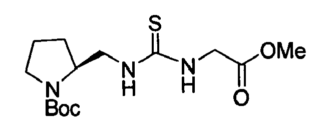

Yield 85%; light yellow oil; [α]_{D}= -1.8 (*c* = 1.0, CH₂Cl₂); ¹H NMR (200 MHz, CDCl₃) δ 7.86 (1H, br H, NH), 7.12 (0.6H, br s, NH), 6.97 (0.4H, br s, NH), 4.39-4.11 (2H, m, NCH and NCHH), 3.96-3.62 (2H, m, 2 x NCHH), 3.63 (3H, s, OCH₃), 3.52-3.30 (1H, m, NCHH), 3.30-3.08 (2H, m, 2 x NCHH), 1.95-1.61 (4H, m, 4 x CHH), 1.33 [9H, s, C(CH₃)₃]; ¹³C NMR (50 MHz, CDCl₃) δ 182.0 (C=S), 170.1 (C=O), 156.4 (OCONH), 79.9 [C(CH₃)₃], 56.0 (NCH), 51.9 (OCH₃), 46.8 (NCH₂), 46.6 (NCH₂),45.7 (NCH₂), 29.2 [CH₂), 28.1 [C(CH₃)], 23.4 (CH₂); MS (ESI) 332 (M+H⁺, 100%).

### (S)-tert-Butyl-2-{[3-((S)-1-methoxy-1-oxo-3-phenylpropan-2-yl)thioureido]methyl}pyrrolidine-1-carboxylate (2d)

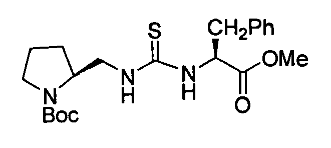

Yield 89%; light yellow oil; [α]_{D}= +2.8 (*c* = 1.0, CHCl₃); ¹H NMR (200 MHz, CDCl₃) δ 7.44-7.09 (5H, m, ArH), 5.21 (1H, br s, NCH), 4.11-3.91 (1H, m, NCH), 3.70 (3H, s, OCH₃), 3.57-3.05 (6H, m, 4 x NCHH and 2 x CHHPh), 2.13-1.56 (4H, m, 4 x CHH), 1.47 [9H, s, C(CH₃)₃]; ¹³C NMR (50 MHz, CDCl₃) δ 181.5 (181.3) (C=S), 172.5 (172.9) (C=O), 155.4 (OCONH), 136.0 (Ar), 129.1 (Ar), 128.2 (Ar), 126.6 (Ar), 80.1 [C(CH₃)₃], 60.1 (NCH), 56.0 (56.3) (NCH), 52.0 (OCH₃), 46.6 (46.6) (NCH₂), 46.5 (46.5) (NCH₂), 37.5 (37.4) (CH₂), 29.4 (29.4) (CH₂), 28.2 [C(CH₃)], 23.5 (CH₂); MS (ESI) 444 (M+Na, 53%), 422 (M+H⁺, 100%).

### (S)-tert-Butyl-2-{[3-(S)-2-methoxy-2-oxo-1-phenylethyl)thioureido]methyl}pyrrolidine-1-carboxylate (2e)

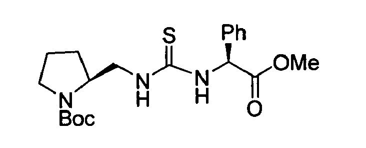

Yield 83%; light yellow oil; [α]_{D}= -4.8 (*c* = 1.0, CH₂Cl₂); ¹H NMR (200 MHz, CDCl₃) δ 7.62-7.21 (7H, m, ArH and 2 x NH), 5.29-5.02 (1H, m, NCH), 4.11-3.86 (1H, m, NCH), 3.84-3.64 (3H, s, OCH₃), 3.57-3.17 (4H, m, 4 x NCHH), 2.05-1.61 (4H, m, 4 x CHH), 1.43 [9H, s, C(CH₃)₃]; ¹³C NMR (50 MHz, CDCl₃) δ 184.5 (181.5) (C=S), 171.3 (172.7) (C=O), 156.9 (155.0) (OCONH), 135.8 (133.1) (Ar), 128.7 (128.8) (Ar), 128.5 (Ar), 126.7 (127. 6) (Ar), 80.3 (80.4) [C(CH₃)₃], 62.3 (60.4) (NCH), 56.0 (56.4) (NCH), 52.7 (52.7) (OCH₃), 46.8 (46.9) (NCH₂), 46.0 (46.1) (NCH₂), 29.6 (CH₂), 28.3 [C(CH₃)], 23.7 (CH₂); MS (ESI) 408 (M+H⁺, 100%).

### (S)-tert-Butyl-2-{[3-((S)-1-methoxy-3,3-dimethyl-1-oxobutan-2-yl)thioureido]methyl}pyrrolidine-1-carboxylate (2f)

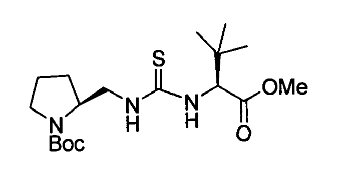

Yield 84%; light yellow oil; [α]_{D}= -8.9 (*c* = 1.0, CH₂Cl₂); ¹H NMR (200 MHz, CDCl₃) δ 8.04 (1H, br H, NH), 7.13 (0.5H, br s, NH), 6.37 (0.5H, m, NH), 4.97-4.55 (1H, m, NCH), 4.01-3.64 (2H, m, NCH and NCHH), 3.60 (3H, s, OCH₃), 3.51-3.32 (1H, m, NCHH), 3.31-3.08 (2H, m, 2 x NCHH), 1.92-1.57 (4H, m, 4 x CHH), 1.35 [9H, s, C(CH₃)₃], 0.93 [9H, s, C(CH₃)₃]; ¹³C NMR (50 MHz, CDCl₃) δ 181.9 (182.1) (C=S), 171.4 (171.6) (C=O), 156.8 (OCONH), 80.1 (80.0) [C(CH₃)₃], 65.7 (NCH), 56.0 (NCH), 51.5 (OCH₃), 46.7 (NCH₂), 46.6 (NCH₂), 34.8 [C(CH₃)₃], 29.2 (CH₂), 28.2 [C(CH₃)], 26.5 [C(CH₃)], 23.6 (CH₂);MS (ESI) 388 (M+H⁺, 100%).

### (S)-tert-Butyl-2-{[3-((R)-2-methoxy-2-oxo-1-phenylethyl)thioureido]methyl}pyrrolidine-1-carboxylate (2g)

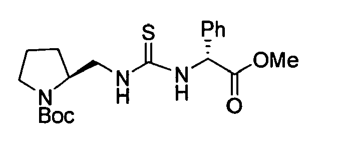

Yield 82%; light yellow solid; mp 67-69 °C; [α]_{D}= -15.3 (*c* = 1.0, CH₂Cl₂); ¹H NMR (200 MHz, CDCl₃) δ 7.99 (1H, br H, NH), 7.51-7.21 (6H, m, ArH and NH), 6.21-5.93 (1H, br m, NCHPh), 4.03-3.77 (1H, m, NCH), 3.68-3.59 (1H, m, NCHH), 3.66 (3H, s, OCH₃), 3.51-3.39 (1H, m, NCHH), 3.33-3.14 (2H, m, 2 x NCHH), 1.99-1.61 (4H, m, 4 x CHH), 1.38 [9H, s, C(CH₃)₃]; ¹³C NMR (50 MHz, CDCl₃) δ 181.0 (180.9) (C=S), 171.2 (171.0) (C=O), 156.3 (156.0) (OCONH), 135.9 (135.7) (Ar), 128.6 (Ar), 128.3 (Ar), 127.5 (127.4) (Ar), 80.1 [C(CH₃)₃], 60.2 (60.7) (NCH), 56.4 (55.9) (NCH), 52.5 (52.5) (OCH₃), 46.7 (46.7) (NCH₂), 46.6 (NCH₂), 29.4 (CH₂), 28.2 [C(CH₃)], 23.6 (CH₂); MS (ESI) 408 (M+H⁺, 100%).

### B. General procedure for the ring-closure step under acidic conditions

Thiourea **2** (0.5 mmol) was put in a pressure vessel and HCl 6N in methanol (3 mL) and AcOH (5 mL) were added. The reaction mixture was heated to 100 °C and left stirring at that temperature for 3 h. It was then cooled to room temperature and the pH of the mixture was adjusted to 8 with an aq. solution of NaHCO₃ (10%). The resulting mixture was extracted with CH₂Cl₂ (3 x 10 mL). The combined organic layers were dried over Na₂SO₄ and the solvent was evaporated to afford the desired product.

Following the above process, the following compounds are prepared:

### (S)-6-Phenyl-3-[(S)-pyrrolidin-2-ylmethyl]-2-thioxotetrahydropyrimidin-4(1H)-one (3a)

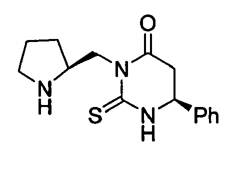

Yield 88%; light yellow solid; mp 88-90 °C; [α]_{D}= -35.7 (*c* = 1.0, CH₂Cl₂); ¹H NMR (200 MHz, CDCl₃) δ 7.33-7.14 (5H, m, ArH), 5.21 (2H, br m, 2 x NH), 4.43 (1H, ddd, *J* = 14.9, 8.3 and 3.7 Hz, NCH), 3.81-3.59 (2H, m, NCH and NCHH), 3.49-3.34 (1H, m, NCHH), 3.24 (1H, dd, *J* = 14.0 and 3.6 Hz, NCHH), 2.97-2.58 (3H, m, NCHH andCHHCO), 1.79-1.54 (3H, m, 3 x CHH), 1.37-1.20 (1H, m, CHH); ¹³C NMR (50 MHz, CDCl₃) δ 184.0 (183.7) (C=S), 174.0 (174.6) (C=O), 135.0 (135.0) (Ar), 129.3 (129.2) (Ar), 128.7 (Ar), 127.4 (Ar), 60.8 (60.5) (NCH), 57.0 (57.0) (NCH), 46.0 (45.8) (NCH₂), 44.9 (NCH₂), 37.3 (COCH₂), 29.4 (29.5)(CH₂), 25.0 (24.9) (CH₂); MS (ESI) 290 (M+H⁺, 100%).

### (S)-3-(Pyrrolidin-2-ylmethyl)-2-thioxotetrahydropyrimidin-4(1H)-one (3b)

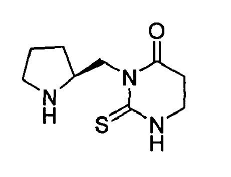

Yield 82%; light yellow oil; [α]_{D}= -66.9 (*c* = 1.0, CH₂Cl₂); ¹H NMR (200 MHz, CDCl₃) δ 3.95 (1H, dd, *J* = 10.8 and 8.2 Hz, CHHNHC=S), 3.79 (1H, dddd, *J* = 8.3, 8.2, 5.2 and 3.4 Hz, NCH), 3.70-3.55 (2H, m, 2 x NCHH), 3.54-3.35 (2H, m, 2 x NCHH), 3.21 (1H, ddd, *J* = 11.3, 9.0 and 3.6 Hz, NCHHCH₂CH₂), 2.72 (1H, br s, NH), 2.41 (2H, dd, *J* = 8.7 and 5.5 Hz, COCH₂), 2.12-1.79 (4H, m, 4 × CHH); ¹³C NMR (50 MHz, CDCl₃) δ 169.3 (C=S), 153.8 (C=O), 58.4 (NCH), 47.0 (NCH₂), 45.2 (NCH₂), 42.7 (NCH₂), 31.5 (COCH₂), 30.1 (CH₂), 26.0 (CH₂); MS (ESI) 198 (M+H-O⁺, 100%), 180 (M+H-H₂S⁺, 92%).

### (S)-3-(Pyrrolidin-2-ylmethyl)-2-thioxoimidazolidin-4-one (3c)

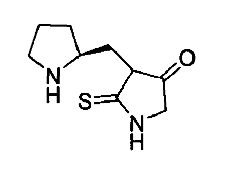

Yield 76%; oil; [α]_{D}= -66.9 (c = 1, CH₂Cl₂); ¹H NMR (200 MHz, CDCl₃) δ 4.43 (2H, m, NCH and NCHH), 3.84-3.78 (1H, m, NCHH), 3.69-3.51 (3H, m, 3 x NCHH), 3.35-3.27 (1H, m, NCHH), 2.31-1.90 (4H, m, 4 x CHH); MS (ESI) 200 (M+H⁺, 11%), 184 (M+H-O⁺, 100%), 166 (M+H-H₂S⁺, 92%).

### (S)-5-Benzyl-3-[(S)-pyrrolidin-2-ylmethyl)-2-thioxoimidazolidin-4-one (3d)

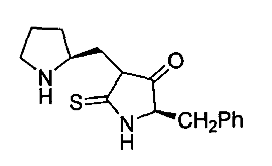

Yield 80%; light yellow solid; mp 96-98 °C; [α]_{D}= -16.8 (*c* = 1, CHCl₃); ¹H NMR (200 MHz, CDCl₃) δ 7.49-6.99 (5H, m, ArH), 6.17-5.50 (2H, br m, 2 x NH), 4.56-4.32 (1H, m, NCH), 3.93-3.57 (2H, m, NCH and NCHH), 3.56-3.35 (1H, m,NCHH), 3.26 (1H, dd, *J* = 14.0 and 4.0 Hz, CHHPh), 3.11-2.59 (3H, m, CHHPh and 2 x NCHH), 2.05-1.52 (4H, m, 4 x CHH); ¹³C NMR (50 MHz, CDCl₃) δ 183.9 (183.6) (C=S), 173.9 (174.5) (C=O), 134.9 (Ar), 129.3 (Ar), 128.8 (Ar), 127.4 (Ar), 60.9 (60.6) (NCH), 57.2 (NCH), 45.8 (45.9) (NCH₂), 44.6 (NCH₂), 37.3 (CH₂), 29.2 (CH₂), 24.8 (24.7) (CH₂); MS (ESI) 290 (M+H⁺, 100%).

### (S)-5-Phenyl-3-[(S)-pyrrolidin-2-ylmethyl)-2-thioxoimidazolidin-4-one (3e)

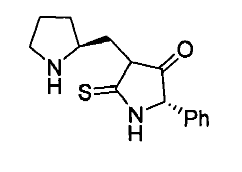

Yield 85%; light yellow solid; mp 166-168 °C; [α]_{D}= +36.8 (c = 1, CH₂Cl₂); ¹H NMR (200 MHz, CDCl₃) δ 7.51-7.07 (5H, m, ArH), 6.78 (2H, br s, 2 x NH), 5.29-5.08 (1H, m, NCH), 4.21-3.44 (3H, m, NCH and 2 x NCHH), 3.35-3.04 (1H, m, NCHH), 2.99-2.50 (1H, m, NCHH), 2.07-1.59 (3H, m, 3 x CHH), 1.51-1.27 (1H, m, CHH); ¹³C NMR (50 MHz, CDCl₃) δ 182.4 (C=S), 172.0 (C=O), 136.4 (Ar), 129.0 (Ar), 128.6 (Ar), 126.9 (Ar), 73.5 (NCH), 58.1 (NCH), 45.2 (NCH₂), 43.0 (NCH₂), 28.9 (CH₂), 25.5 (CH₂); MS (ESI) 276 (M+H⁺, 100%).

### (S)-5-tert-Butyl-3-[(S)-pyrrolidin-2-ylmethyl)-2-thioxoimidazolidin-4-one (3f)

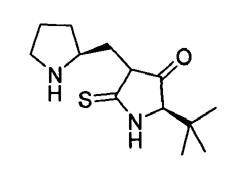

Yield 72%; light yellow oil; [α]_{D}= -8.8 (*c* = 1, CH₂Cl₂); ¹H NMR (200 MHz, CDCl₃) δ 5.30 (2H, br s, 2 x NH), 3.90-3.67 (3H, m, 2 x NCH and NCHH), 3.64-3.40 (1H, m, NCHH), 3.08-2.94 (1H, m, NCHH), 2.91-2.76 (1H, m, NCHH), 1.95-1.58 (3H, m, 3 x CHH), 1.52-1.32 (1H, m, CHH), 1.00 [9H, s, C(CH₃)₃]; ¹³C NMR (50 MHz, CDCl₃) δ 184.0 (184.2) (C=S), 174.0 (173.5) (C=O), 67.8 (67.9) (NCH), 56.9 (NCH), 46.2 (46.1) (NCH₂), 45.0 (45.1) (NCH₂), 35.4 [C(CH₃)₃], 29.6 (29.5) (CH₂), 25.5 [C(CH₃)], 25.2 (25.1) (CH₂); MS (ESI) 256 (M+H⁺, 100%).

### (R)-5-Phenyl-3-[(S)-pyrrolidin-2-ylmethyl)-2-thioxoimidazolidin-4-one (3g)

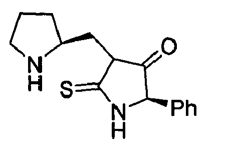

Yield 82%; light yellow solid; mp 145-147 °C; [α]_{D}= +23.8 (c = 0.5, CH₂Cl₂); ¹H NMR (200 MHz, CDCl₃) δ 7.52-7.06 (5H, m, ArH), 6.17 (2H, br s, 2 x NH), 5.27-5.17 (1H, m, NCH), 4.10-3.89 (1H, m, NCH), 3.80-3.47 (2H, m, 2 x NCHH), 3.35-3.04 (1H, m, NCHH), 3.04-2.65 (1H, m, NCHH), 2.07-1.54 (3H, m, 3 x CHH), 1.52-1.34 (1H, m, CHH); ¹³C NMR (50 MHz, CDCl₃) δ 182.3 (C=S), 172.0 (C=O), 133.4 (Ar), 129.0 (Ar), 128.7 (Ar), 126.9 (Ar), 73.5 (NCH), 58.1 (NCH), 45.2 (NCH₂), 43.0 (NCH₂), 28.9 (CH₂), 25.4 (CH₂); MS (ESI) 276 (M+H⁺, 100%).

### C. General procedure for the ring-closure step under basic conditions

Boc-protected thiourea (0.33 mmol) was dissolved in acetonitrile (3 mL) in a pressure vessel. Triethylamine (0.06 mL, 0.40 mmol) and lithium perchlorate (4.5 mg, 0.04 mmol) were added. The reaction mixture was heated at 85 °C and left stirring at that temperature for 2 hours. It was then cooled to room temperature and the solvents were evaporated under vacuo. The resulting crude was diluted with dichloromethane and washed with water (2 x 10 mL) and brine (1 x 10 mL), dried over Na₂SO₄ and the solvent was evaporated to afford the desired product.

Following the above process, the following compound is prepared:

### (S)-tert-Butyl 2-{[(S)-6-oxo-4-phenyl-2-thioxotetrahydropyrimidin-1(2H)-yl]methyl}pyrrolidine-1-carboxylate (4a)

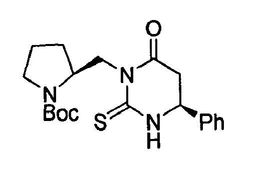

Yield 69%; light yellow solid; mp 93-95 °C; [α]_{D}= + 48.4 (*c* = 0.5, CH₂Cl₂); ¹H NMR (200 MHz, CDCl₃) δ 8.02 (0.5H, br s, NH), 7.79 (0.5H, br s, NH), 7.52-7.23 (5H, m, ArH), 4.50-4.21 (1H, m, NCH), 4.16-3.83 (1H, m, NCH), 3.80-3.49 (1H, m, NCHH), 3.47-3.15 (4H, m, 4 x NCHH), 3.14-2.89 (1H, m, NCHH), 2.09-1.58 (4H, m, 4 x CHH), 1.41 [9H, s, C(CH₃)₃]; MS (ESI) 390 (M+H⁺, 100%).

## Claims

1. A compound according to the formula I 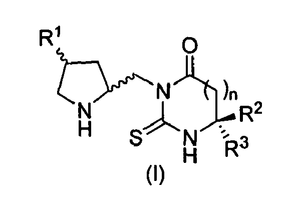 wherein
R¹ is H, OH, O-alkyl, OSi(CH₃)₃, OSiC(CH₃)₃(CH₃)₂,
R² is H, alkyl, aryl,
R³ is H, alkyl, aryl,
provided that when R² is H, CH₃, then R³ is other than H, CH₃,
n is 0 or 1,
their stereoisomers,
or their salts thereof.

2. A compound according to claim 1, wherein R¹ is H.

3. A compound according to claim 2 wherein R³ is H and R² is C2-C5 alkyl, phenyl.

4. A compound according to anyone of claims 1 to 3, wherein n is 1.

5. A compound according to claim 4, wherein
R¹=H, R²=Ph, R³=H.

6. A compound according to claim 4, wherein
R¹=H, R²= C(CH₃)₃, R³=H.

7. A compound according to claim 1 to 3, wherein n is 0.

8. A compound according to claim 7, wherein
R¹ = H, R² = Ph, R³ = H.

9. A compound according to claim 7, wherein
R¹ = H, n = 0, R² = C(CH₃)3, R³ = H.

10. A process for the preparation of a compound according to anyone of claims 1 to 9 comprising the reaction of 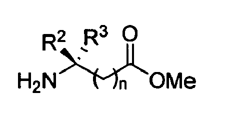 with 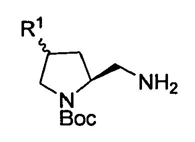 to prepare thioureas 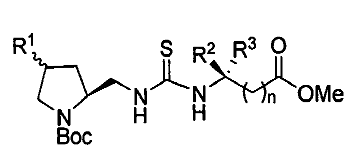 which are converted to 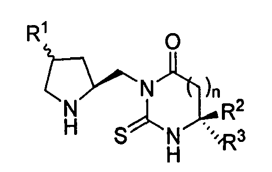 under acidic or basic conditions.
